(19) **Europäisches Patentamt / European Patent Office / Office européen des brevets**

(11) **EP 4 628 025 A1**

(12) **EUROPEAN PATENT APPLICATION**
published in accordance with Art. 153(4) EPC

(43) Date of publication:
**08.10.2025 Bulletin 2025/41**

(21) Application number: **23897422.4**

(22) Date of filing: **09.11.2023**

(51) International Patent Classification (IPC):
**A61B 10/00** (2006.01)    **A61B 1/24** (2006.01)
**A61C 19/04** (2006.01)    **A61B 1/00** (2006.01)
**A61B 5/00** (2006.01)    **G16H 50/20** (2018.01)

(52) Cooperative Patent Classification (CPC):
**G16H 50/20; A61B 1/00009; A61B 1/24;**
**A61B 5/0075; A61B 5/0088; A61B 5/4547;**
**A61B 5/4552; A61B 10/00; A61C 19/04**

(86) International application number:
**PCT/JP2023/040358**

(87) International publication number:
**WO 2024/116769 (06.06.2024 Gazette 2024/23)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **28.11.2022   JP 2022189573**

(71) Applicant: **Panasonic Intellectual Property**
**Management Co., Ltd.**
**Kadoma-shi, Osaka 571-0057 (JP)**

(72) Inventors:
• **ICHIMURA Ryo**
**Kadoma-shi, Osaka 571-0057 (JP)**
• **NUNOMURA Mahito**
**Kadoma-shi, Osaka 571-0057 (JP)**

• **NARITA Kenji**
**Kadoma-shi, Osaka 571-0057 (JP)**
• **OIKAWA Kazuma**
**Kadoma-shi, Osaka 571-0057 (JP)**
• **SUVARNADAS Rinoy**
**Trivandrum 695012 (IN)**
• **RAJAMOHANAN Deepak**
**Trivandrum 695012 (IN)**
• **RADHAKRISHNAN Rahul**
**Trivandrum 695012 (JP)**
• **KARUVAPPURAKKAL Nikhil Vishnu**
**Trivandrum 695012 (JP)**
• **NARAYANAN Subhash**
**Trivandrum 695012 (IN)**

(74) Representative: **Müller-Boré & Partner**
**Patentanwälte PartG mbB**
**Friedenheimer Brücke 21**
**80639 München (DE)**

(54) **PERIODONTAL DISEASE DETECTION SYSTEM, PERIODONTAL DISEASE DETECTION METHOD, AND PERIODONTAL DISEASE DETECTION PROGRAM**

(57)    A periodontal disease detection system (1) includes a periodontal disease determination device (100) and a measurement device. The periodontal disease determination device (100) has a position adjustment unit (112) which acquires an image of the measurement device set in the oral cavity by the user from an imaging unit, and displays the image on a display unit to allow the user to adjust the position of the measurement device. Further, the periodontal disease determination device (100) includes a light emission control unit (113) which transmits to the measurement device a light emission control instruction for irradiating light from a light source provided in the measurement device. Further, the periodontal disease determination device (100) includes a measurement data acquisition unit (114) that acquires information on an intensity of reflected light acquired by the measurement device as measurement data. Furthermore, the periodontal disease determination device (100) includes a periodontal disease determination unit (115) that calculates information indicating the degree of periodontal disease of the user based on the measurement data.

# FIG. 4

**Description**

TECHNICAL FIELD

The present disclosure relates to a periodontal disease detection system, a periodontal disease detection method, and a periodontal disease detection program.

BACKGROUND ART

Conventionally, a method for detecting periodontal disease in the oral cavity based on reflected light from irradiated light (Non-patent literature 1) and a system for detecting periodontal disease in the oral cavity using the method have been proposed. Patent Literature 1 discloses a periodontal disease detection system by irradiation of light. In the periodontal disease detection system disclosed in Patent Literature 1, the condition of periodontal disease in the oral cavity is specified and highlighted based on the reflected light, which is irradiated in the oral cavity by an oral cavity camera including a light source and an image sensor array.

CITATION LIST

PATENT LITERATURE

Patent Literature 1: Inernational Publication No.2015/069704

NON-PATENT LITERATURE

Non-patent Literature 1: Prasanth Chandra Sekhar et al, "Discrimination of periodontal diseases using diffuse reflectance spectral intensity ratios," Journal of Biomedical Optics 17 (2), February 2012, P.027001-1-P.027001-9

SUMMARY OF INVENTION

In the periodontal disease detection system disclosed in Patent Literature 1, an image acquired by the oral cavity camera is displayed on a display, and a user positions the oral cavity camera based on the image. However, since the teeth and gums in the oral cavity have similar shapes in the image acquired by the oral cavity camera, it is sometimes difficult for a user to determine the accurate measurement position in the periodontal disease detection system.

The present disclosure has been made in view of such problems in the conventional technology. An object of the present disclosure is to provide a periodontal disease detection system capable of accurately determining the position of a periodontal part to be detected.

A periodontal disease detection system according to an aspect of the present disclosure provides a periodontal disease detection system including a periodontal disease determination device and a measurement device, in which the periodontal disease determination device includes: an input information acquisition unit that acquires information inputted by a user on a periodontal part to be measured; a position adjustment unit that acquires an image of a measurement device set in the oral cavity by the user from an imaging unit, and displaying the image on a display unit to allow the user to adjust the position of the measurement device; a light emission control unit that transmits to the measurement device a light emission control instruction for irradiating light from a light source provided in the measurement device; a measurement data acquisition unit that acquires information on an intensity of reflected light acquired by the measurement device as measurement data; and a periodontal disease determination unit that calculates information indicating the degree of periodontal disease of the user based on the measurement data; and in which the measurement device includes: a light source that irradiates light based on a light emission control instruction from a light emission control unit; and a reflected light acquisition unit that acquires reflected light of the irradiated light.

A periodontal disease detection method according to another aspect of the present disclosure is a periodontal disease detection method executed by a computer, including: acquiring information, inputted by a user, on a periodontal part to be measured; acquiring an image of a measurement device set in the oral cavity by the user to allow the user to adjust the position of the measurement device; displaying the image on a display unit; transmitting to the measurement device a light emission control instruction for irradiating light from a light source provided in the measurement device; acquiring information on an intensity of reflected light, which is irradiated from the light source and is acquired by a reflected light acquisition unit provided in the measurement device, as measurement data; and calculating information indicating the degree of periodontal disease of the user based on the measurement data.

A periodontal disease detection program according to another aspect of the present disclosure causes a computer to execute the steps of acquiring information, inputted by a user, on a periodontal part to be measured; acquiring an image of a measurement device set in the oral cavity by the user to allow the user to adjust the position of the measurement device; displaying the image on a display unit; transmitting to the measurement device a light emission control instruction for irradiating light from a light source provided in the measurement device; acquiring information on an intensity of reflected light, which is irradiated from the light source and is acquired by a reflected light acquisition unit provided in the measurement device, as measurement data; and calculating information indicating the degree of periodontal disease of the user based on the measurement data.

## BRIEF DESCRIPTION OF DRAWINGS

[Fig. 1] Fig. 1 illustrates the external appearance of a periodontal disease detection system according to the present embodiment.

[Fig. 2] Fig. 2 is a block diagram illustrating the configuration of a periodontal disease detection system according to the present embodiment.

[Fig. 3A] Fig. 3A is a diagram for explaining a light source and a reflected light acquisition unit according to the present embodiment.

[Fig. 3B] Fig. 3B is a graph for explaining a wavelength of irradiated light and reflected light according to the present embodiment.

[Fig. 4] Fig. 4 is a block diagram illustrating a functional configuration of the periodontal disease determination device according to the present embodiment.

[Fig. 5] Fig. 5 is a diagram illustrating user information according to the present embodiment.

[Fig. 6A] Fig. 6A illustrates an example of an input screen for user information according to the present embodiment.

[Fig. 6B] Fig. 6B illustrates an example of an input screen for tooth position information according to the present embodiment.

[Fig. 6C] Fig. 6C illustrates an example of an input screen for information on a gingival region according to the present embodiment.

[Fig. 6D] Fig. 6D illustrates an example of an input screen for information on a gingival region according to the present embodiment.

[Fig. 6E] Fig. 6E illustrates centrifugal and mesial motion in the oral cavity.

[Fig. 7A] Fig. 7A illustrates an example of a screen displayed on a display unit of a periodontal disease determination device according to the present embodiment.

[Fig. 7B] Fig. 7B illustrates an example of a screen displayed on a display unit of a periodontal disease determination device according to the present embodiment.

[Fig. 8] Fig. 8 is a graph for explaining the relationship between a wavelength of reflected light and a diffuse reflection intensity.

[Fig. 9] Fig. 9 illustrates an example of a screen displayed on the display unit of the periodontal disease determination device according to the present embodiment.

[Fig. 10] Fig. 10 is a flowchart illustrating an example of processing of the periodontal disease detection system according to the present embodiment.

[Fig. 11] Fig. 11 is a flowchart illustrating an example of processing related to acquisition of input information in the periodontal disease detection system according to the present embodiment.

[Fig. 12] Fig. 12 is a flowchart illustrating an example of position adjustment processing in the periodontal disease detection system according to the present embodiment.

[Fig. 13A] Fig. 13A illustrates an example of a user information input screen according to another embodiment.

[Fig. 13B] Fig. 13B illustrates an example of a user information input screen according to another embodiment.

## DESCRIPTION OF EMBODIMENTS

Hereinafter, an embodiment will be described in detail with reference to the drawings. However, detailed description more than necessary may be omitted. For example, a detailed description of a well-known matter or a duplicate description of substantially identical configurations may be omitted. The accompanying drawings and the following description are provided for a person skilled in the art to fully understand the present disclosure, and are not intended to limit the subject matter described in the claims.

(Periodontal Disease Detection System 1)

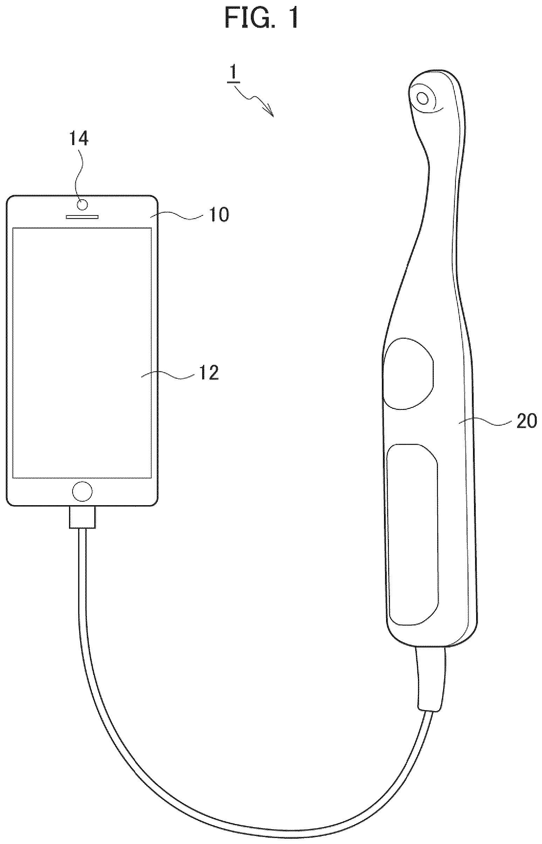

With reference to FIG. 1, the configuration of a periodontal disease detection system 1 according to the present embodiment will be described. FIG. 1 illustrates the external appearance of the periodontal disease detection system 1 according to the present embodiment. In the present embodiment, the periodontal disease detection system 1 includes a user terminal 10 and a measurement device 20.

In the example illustrated in FIG. 1, the user terminal 10 and the measurement device 20 of the periodontal disease detection system 1 are connected via a communication cable. The measurement device 20 is a device for acquiring information on periodontal disease in the oral cavity of a user. The measurement device 20 operates according to control by the user terminal 10, and transmits acquired data to the user terminal 10. The present embodiment is not limited to a configuration in which the user terminal 10 and the measurement device 20 are connected by a communication cable, and a configuration in which the user terminal 10 and the measurement device 20 can communicate by wireless communication may be adopted. The user terminal 10 includes a display unit 12 and an imaging unit 14.

(Configuration of User Terminal 10)

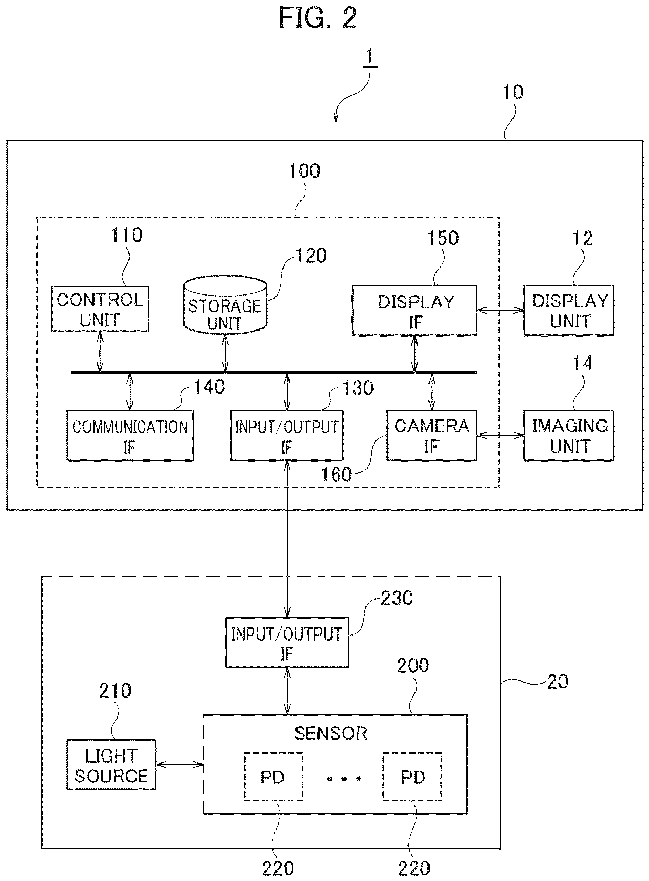

Next, with reference to FIG. 2, a configuration example of the periodontal disease detection system 1 according to the present embodiment will be described. FIG. 2 is a block diagram illustrating the configuration of the user terminal 10 and the measurement device 20 included in the periodontal disease detection system 1 according to the present embodiment.

As illustrated in FIG. 2, the user terminal 10 includes a periodontal disease determination device 100, the display unit 12, and the imaging unit 14.

The periodontal disease determination device 100 includes a control unit 110, a storage unit 120, and an input/output IF 130 (interface). The periodontal disease determination device 100 may also include a communication IF 140. That is, in the present embodiment, the periodontal disease determination device 100 is configured by a general microcomputer including the control unit 110, the storage unit 120, and the input/output IF 130.

In the present embodiment, multiple information processing functions of the periodontal disease determination device 100 are implemented by software. The periodontal disease determination device 100 includes multiple information processing circuits that function by executing a computer program.

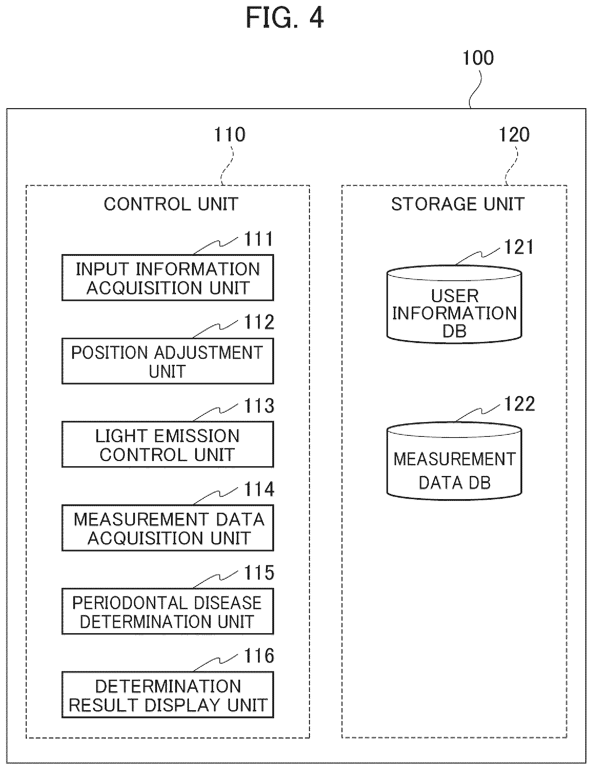

In another configuration, the periodontal disease determination device 100 may be configured by a system LSI (Large Scale Integration) or the like by preparing dedicated hardware for executing each information processing illustrated in FIG. 4. Further, multiple information processing functions may be individually configured by hardware as a system. Details of the control unit 110 and the storage unit 120 will be described below.

The input/output IF 130 is an interface for transmitting and receiving information (data) exchanged between the periodontal disease determination device 100 of the user terminal 10 and the measurement device 20.

The communication IF 140 is an interface for communicating with the outside of the user terminal 10 via a wireless network.

A display IF 150 is an interface for transmitting and receiving information exchanged with the screen of the display unit 12 of the user terminal 10.

A camera IF 160 is an interface for transmitting and receiving information exchanged with the imaging unit 14 and the imaging unit 14 of the user terminal 10.

The display unit 12 corresponds to a display of the user terminal 10. For example, the display unit 12 displays a predetermined screen according to a display instruction from the control unit 110. The display unit 12 has a touch panel function, and the information inputted by the user to the display unit 12 via the touch panel is sent to the control unit 110 or the storage unit 120 via the display IF 150.

The imaging unit 14 corresponds to a camera of the user terminal 10 for acquiring a still image and/or a moving image. The still image and/or the moving image acquired by the imaging unit 14 is sent to the control unit 110 or the storage unit 120 via the camera IF 160.

(Configuration of Measurement Device 20)

Next, a configuration of the measurement device 20 will be described. As illustrated in FIG. 2, the measurement device 20 includes a sensor 200, a light source 210, and an input/output IF 230. The sensor 200 includes a reflected light acquisition unit 220.

The sensor 200 emits light having a plurality of wavelengths from the light source 210 based on a light emission control instruction from the user terminal 10. Further, the sensor 200 transmits the reflected light acquired by the light receiving element (photodiode) to the user terminal 10 via the input/output IF 230.

The light source 210 emits light (white light) based on a light emission control instruction from the light emission control unit 113. In the present embodiment, the light source 210 includes a white LED, and emits white light by light emission control from the user terminal 10. Generally, white light is light obtained by evenly mixing light of a plurality of wavelengths. That is, in the present embodiment, the white light irradiated from the light source 210 has a plurality of wavelengths.

The reflected light acquisition unit 220 acquires information on the reflected light from the white light irradiated from the light source 210, and on the intensity of the reflected light corresponding to a specific wavelength. In the present embodiment, the reflected light may have a plurality of wavelengths. In the present embodiment, the measurement device 20 may have a plurality of the reflected light acquisition units 220 corresponding to the plurality of wavelengths. In the present embodiment, the reflected light acquisition unit 220 includes a PD (PhotoDiode) which is a light receiving element.

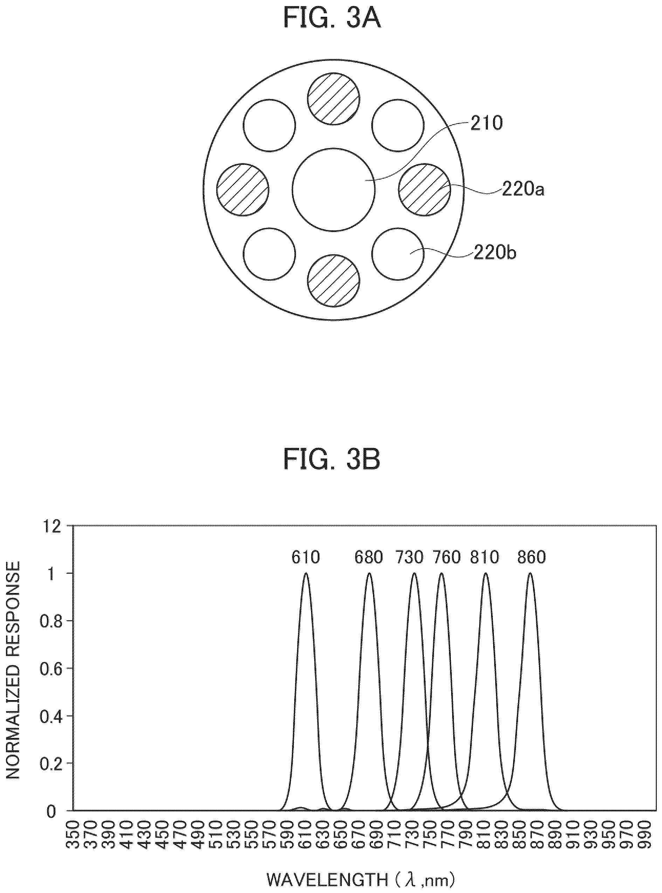

FIG. 3A illustrates a configuration of the light source 210 provided at a tip of the measurement device 20, and the reflected light acquisition unit 220. According to the present embodiment, the light source is located at the center, and two types of reflected light acquisition units 220a and 220b are located around the light source 210, as illustrated in FIG. 3A. The reflected light acquisition unit 220a and the reflected light acquisition unit 220b can acquire reflected light of different wavelengths among the reflected light having a plurality of wavelengths. Hereinafter, when there is no need to describe the reflected light acquisition units 220a and 220b separately, they will simply be referred to as "reflected light acquisition unit 220".

FIG. 3B illustrates the relationship between the wavelengths of the reflected light acquired by the reflected light acquisition unit 220 and a normalized response. FIG. 3B illustrates an example of the normalized response with the wavelengths of 610 nm, 680 nm, 730 nm, 760 nm, 810 nm, and 860 nm. The reflected light acquisition unit 220 in the present embodiment can acquire a predetermined reflected light having a wavelength in the range of 350 nm to 990 nm.

In the present embodiment, for example, the reflected light acquisition unit 220a acquires the reflected light having a wavelength of 620 nm. The reflected light acquisition unit 220b acquires the reflected light having a wavelength of 575 nm. The wavelengths of the reflected light acquired by the reflected light acquisition unit 220a and the reflected light acquisition unit 220b are not limited to those described above. The reflected light acquisition unit 220a and the reflected light acquisition unit 220b may acquire the reflected light having wavelengths other than 620 nm and 575 nm.

The input/output IF 230 is an interface for transmitting and receiving information exchanged between the periodontal disease determination device 100 of the user terminal 10 and the measurement device 20.

(Functions of Periodontal Disease Determination Device 100)

Next, with reference to FIG. 4, a functional configuration of the periodontal disease determination device 100 according to the present embodiment will be described. As illustrated in FIG. 4, the periodontal disease determination device 100 includes an input information acquisition unit 111, a position adjustment unit 112, a light emission control unit 113, a measurement data acquisition unit 114, a periodontal disease determination unit 115, and a determination result display unit 116 as functions. Details of the functions in the control unit 110 will be described below.

The control unit 110 entirely controls the user terminal 10 by operating, for example, an operating system. Furthermore, the control unit 110 operates based on a program stored in the storage unit 120 to execute the functions described above. The program is not limited to that stored in the storage unit 120, but may be stored in a ROM (Read Only Memory) or the like (not illustrated) in the user terminal 10, for example.

The storage unit 120 stores information contained in a user information DB 121 (database), and a measurement data DB 122, as databases, as illustrated in FIG. 4. One or more of the storage units 120 may be provided to store the databases. For example, each of the storage units 120 may be configured to store the databases in separate areas. Alternatively, the databases may be distributed and stored in multiple storage devices arranged at physically separated locations.

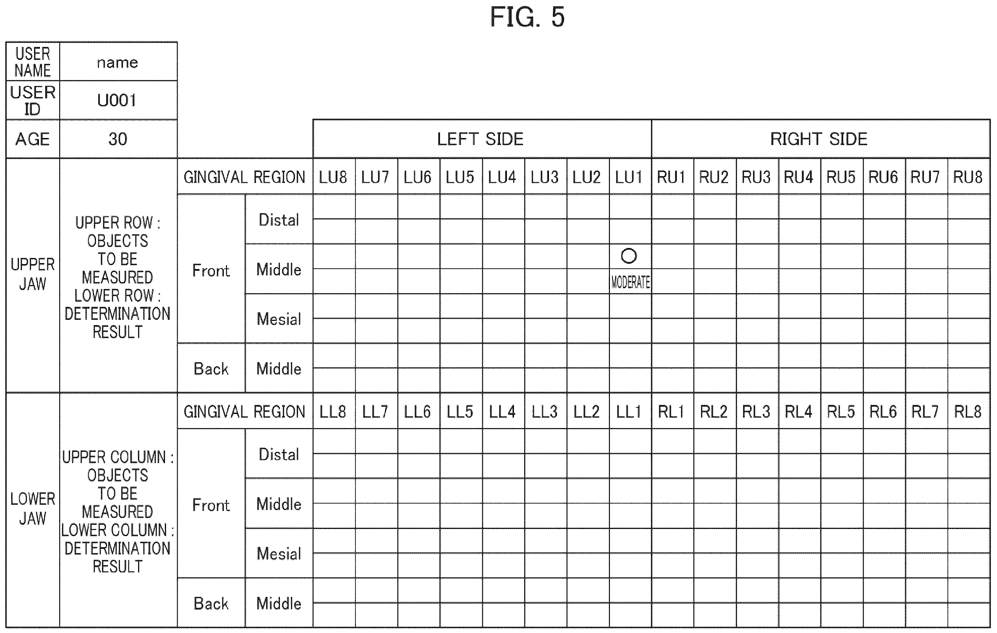

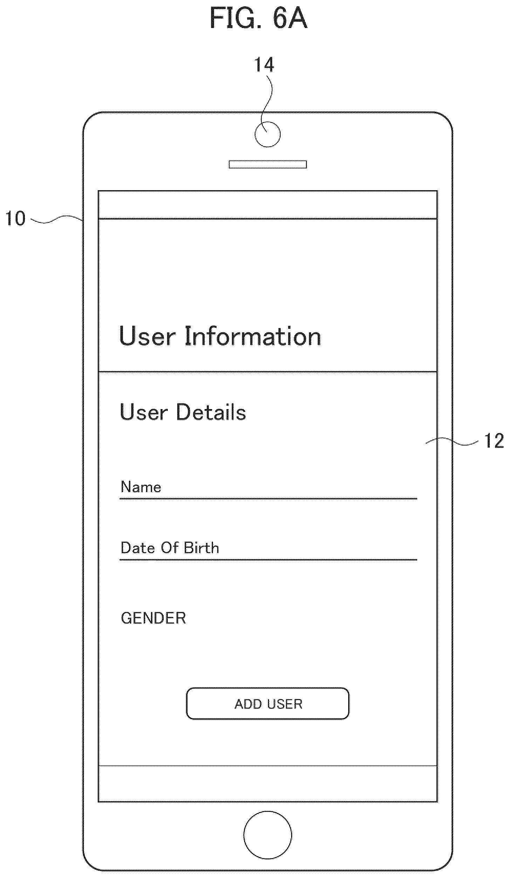

FIG. 5 is a diagram illustrating an example of user information stored in the user information DB 121. Information corresponding to items related to "user name", "user ID", and "age" is stored in the user information. A user inputs information corresponding to an input screen displayed on the display unit 12 of the user terminal 10, as illustrated in FIG. 6A, for example.

Next, the functions of the periodontal disease determination device 100 will be described.

The input information acquisition unit 111 acquires user information, and information related to the periodontal part to be measured, both inputted by a user. Specifically, the input information acquisition unit 111 acquires data inputted by the user to an input screen displayed on the display unit 12 of the user terminal 10.

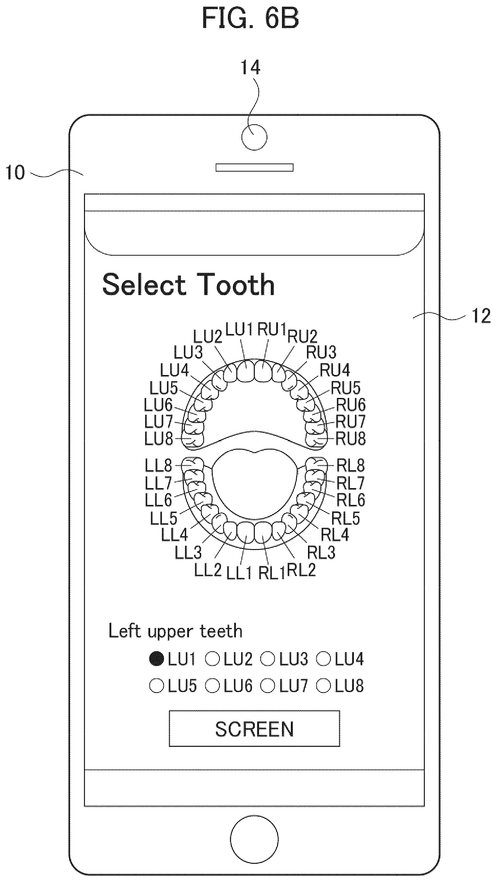

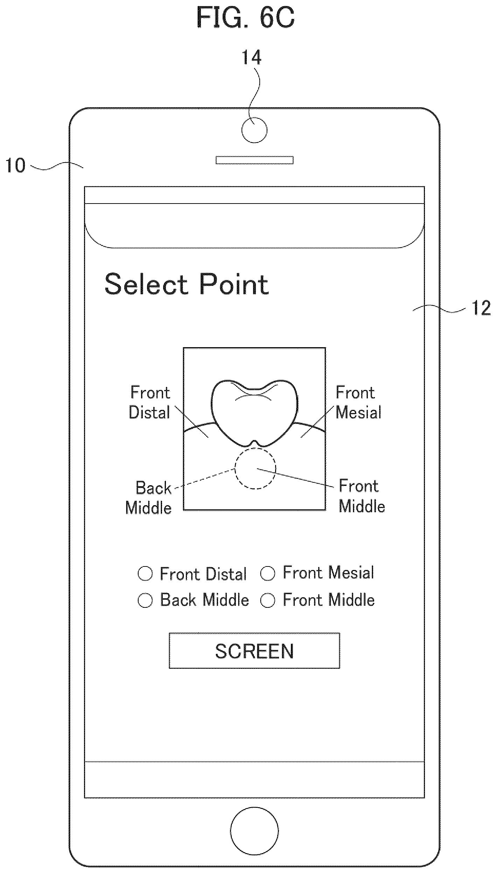

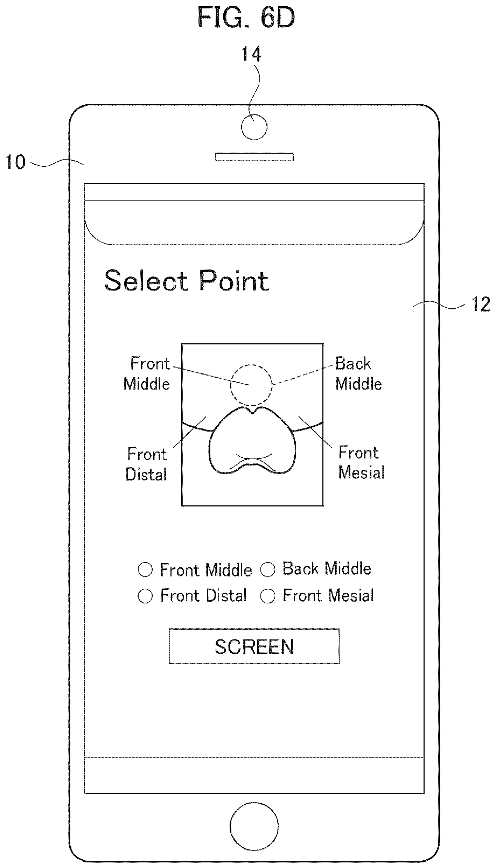

In the present embodiment, the input information acquisition unit 111 acquires input information inputted by the user to the screen illustrated in FIG. 6A. Further, the input information acquisition unit 111 acquires information related to the periodontal part to be measured, based on the information inputted by the user to the input screens illustrated in FIGS. 6B, 6C, and 6D.

FIG. 6B illustrates an input screen for acquiring information related to a position of a tooth to be measured among the information related to the periodontal part to be measured. The user selects a position of a tooth related to the periodontal part to be measured according to the screen illustrated in FIG. 6B.

FIGS. 6C and 6D illustrate input screens for acquiring information related to a gingival region to be measured among the information related to the periodontal part to be measured. FIG. 6C illustrates an input screen for a tooth on the lower jaw side, and FIG. 6D illustrates an input screen for a tooth on the upper jaw side.

In the present embodiment, four patterns of the gingival regions to be measured are provided: "Front Distal", "Front Mesial", "Front Middle", and "Back Middle".

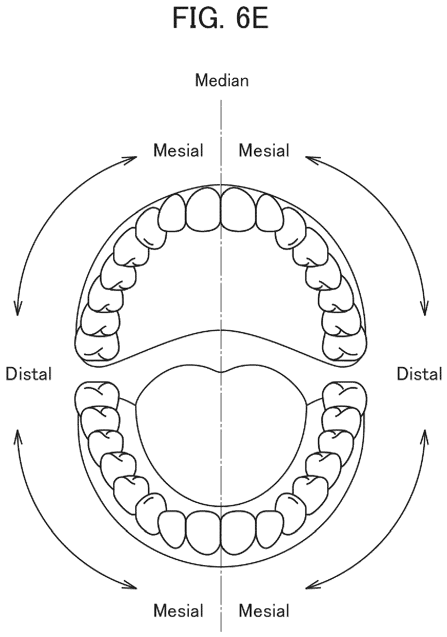

It is noted that a side near a center line (median) indicating a center of upper and lower teeth corresponds to "Mesial", and a side far from the center line corresponds to "Distal", as illustrated in FIG. 6E.

In the examples illustrated in FIGS. 6C and 6D, the left side in the drawings corresponds to "Distal". In other words, the tooth illustrated in FIG. 6C corresponds to the tooth located between "LL1" and "LL8" on the lower jaw side in the example illustrated in FIG. 5. The tooth illustrated in FIG. 6D corresponds to the tooth located between "LU1" and "LU8" on the upper jaw side in the example illustrated in FIG. 5.

The information on the periodontal part to be measured, which is acquired by the input information acquisition unit 111, is stored in the user information DB 121. In the example illustrated in FIG. 5, a case where "Front Middle" of the tooth "LU1" is to be measured is illustrated as an example, and information is stored with "O" in the table. In FIG. 5, information of the measurement data DB 122 is also illustrated as described below. Specifically, in the example illustrated in FIG. 5, information corresponding to the measurement data DB 122 is stored in the lower row corresponding to each of the teeth.

The position adjustment unit 112 acquires an image of a measurement device 10 set in the oral cavity by the user from the imaging unit 14, and displays the image on the display unit 12, to allow the user to adjust the position of the measurement device 20. The user checks whether the measurement device 20 is set in a correct position by looking at the image displayed on the display unit 12.

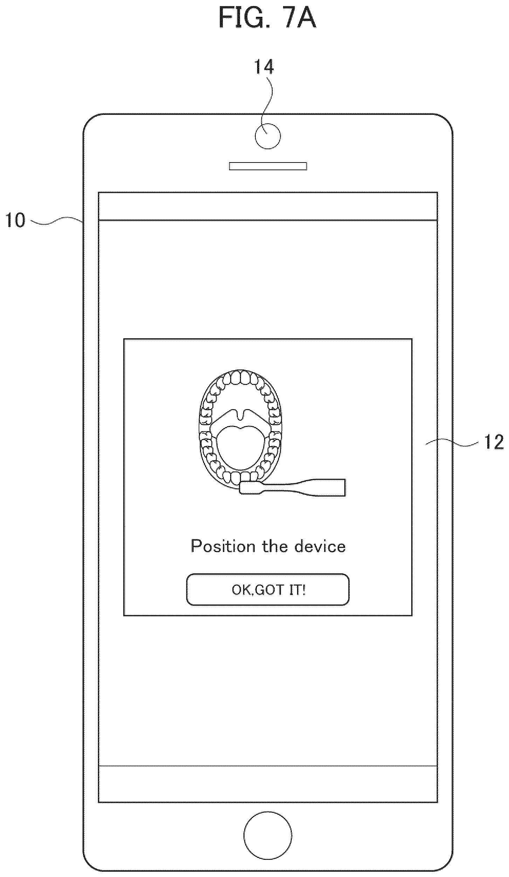

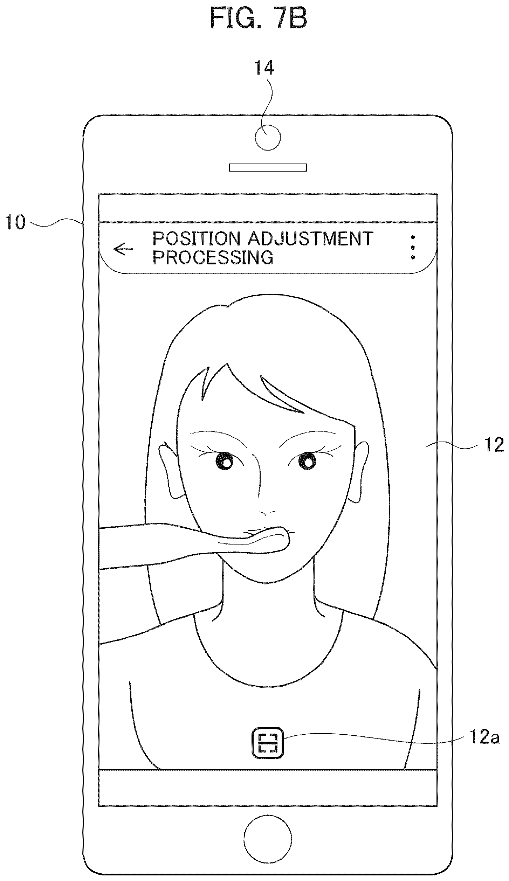

FIGS. 7A and 7B illustrate guide screens to allow a user to adjust the position of the measurement device 20. First, the position adjustment unit 112 displays the image illustrated in FIG. 7A on the display unit 12, and checks whether the user has completed preparation for measurement by the measurement device 20. Then, the position adjustment unit 112 causes the display unit 12 to display the image illustrated in FIG. 7B, and allows the user to perform position adjustment of the measurement device 20. When there is no problem with the position of the measurement device 20, the user presses a start button 12a displayed on the display unit 12 to start measurement by the measurement device 20.

The light emission control unit 113 transmits to the measurement device 20 a light emission control instruction for irradiating light having a plurality of wavelengths from the light source 210 that is provided in the measurement device 20. The light emission control unit 113 transmits a light emission control instruction of the light source 210 to the measurement device 20, whereby white light is irradiated from the light source 210.

The measurement data acquisition unit 114 acquires information on the intensity of the reflected light acquired by the measurement device 20 as measurement data. Specifically, the measurement data acquisition unit 114 acquires information on the intensity of the reflected light acquired by the reflected light acquisition unit 220 that includes a PD (photodiode) as measurement data via the input/output IF 230 and the input/output IF 130.

The periodontal disease determination unit 115 calculates information indicating the degree of periodontal disease of the user based on the measurement data. Specifically, the periodontal disease determination unit 115 calculates information indicating the degree of periodontal disease based on the measurement data transmitted from the measurement device 20. In the present embodiment, information indicating the degree of periodontal disease is calculated based on the intensity of reflected light having a wavelength of 620 nm and the intensity of reflected light having a wavelength of 575 nm. Hereinafter, information obtained by normalizing the intensity of reflected light having a wavelength of 620 nm is expressed as R620, and information obtained by normalizing the intensity of reflected light having a wavelength of 575 nm is expressed as R575.

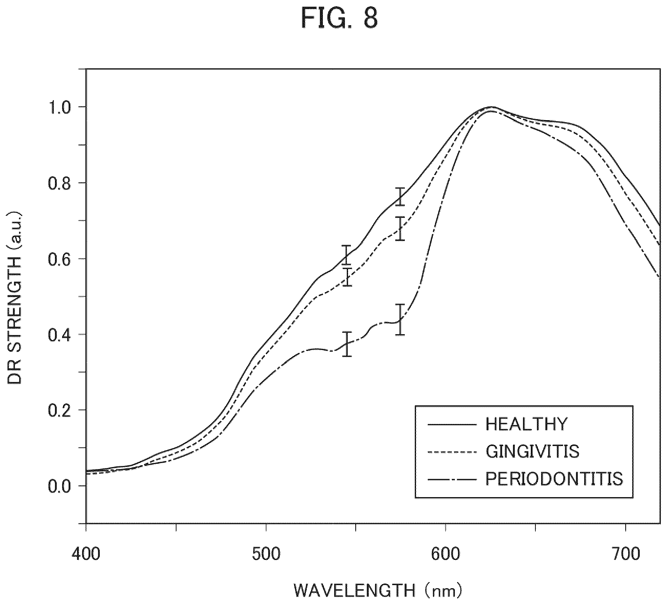

Generally, it is known that the value of R575 decreases as the degree of periodontal disease progresses when R620 has a normalized value of 1. For example, Non-patent literature 1 discloses a diagram illustrating the relationship between the wavelength of reflected light and diffuse reflectance (DR) intensity, as illustrated in FIG. 8. The diffuse reflectance intensity from 400 nm to 750 nm is illustrated for R620 normalized to the maximum intensity, in the example illustrated in FIG. 8. When the reflected light of each wavelength is normalized with R620 having the maximum intensity (1.0), the value of a periodontal disease patient is lower than that of a healthy person, for example, in R575, in the example illustrated in FIG. 8.

In the present embodiment, the periodontal disease determination unit 115 calculates information indicating the degree of periodontal disease according to the ratio (intensity ratio) of R575 when R620 has a normalized value of 1. That is, in the present embodiment, this intensity ratio corresponds to information indicating the degree of periodontal disease. Specifically, the strength ratio is expressed by the following formula (1):

$$\text{Strength ratio} = R620/R575 \ \dots \ (1)$$

In the present embodiment, the periodontal disease determination unit 115 determines the presence or absence of periodontal disease and the degree of periodontal disease based on the calculated strength ratio according to the following formulas (2) to (5):

$$\text{Strength Ratio} \leq 1.30 \ \dots \ (2)$$

$$1.30 < \text{Strength Ratio} \leq 1.44 \ \dots \ (3)$$

$$1.44 < \text{Strength Ratio} \leq 2.27 \ \dots \ (4)$$

$$2.27 < \text{Strength Ratio} \ \dots \ (5)$$

The periodontal disease determination unit 115 determines that there is no problem with periodontal disease when the intensity ratio satisfies formula (2). Furthermore, the periodontal disease determination unit 115 determines that periodontal disease is "mild" when the intensity ratio satisfies formula (3). The periodontal disease determination unit 115 determines that periodontal disease is "moderate" when the intensity ratio satisfies formula (4). Furthermore, the periodontal disease determination unit 115 determines that periodontal disease is "severe" when the intensity ratio satisfies formula (5).

The periodontal disease determination unit 115 stores a determination result in the measurement data DB 122. In FIG. 5, the information of the measurement data DB 122 is stored as the determination result in the lower row of rows corresponding to the teeth, as described above. The example in FIG. 5 illustrates a case where "Front Middle" of the tooth "LU1" is to be measured, and a case where "moderate" is stored as the determination result in the lower row of the table, as an example.

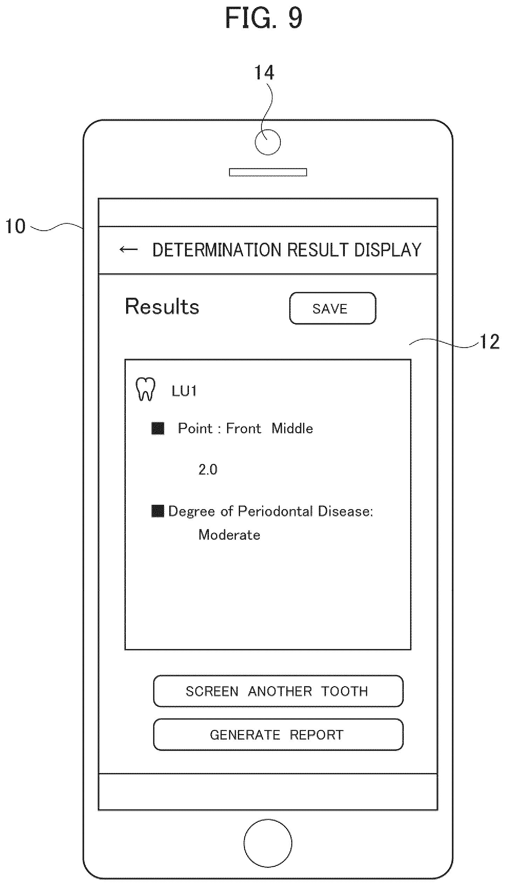

The determination result display unit 116 displays the determination result determined by the periodontal disease determination unit 115 on the display unit 12. FIG. 9 illustrates an example of the determination result of periodontal disease displayed on the display unit 12 of the user terminal 10. As illustrated in FIG. 9, the intensity ratio "2.0", which is the determination result, and the degree of periodontal disease "moderate" are indicated on the display unit 12 for "LU1" as the tooth to be measured, and "Front Middle" as the gingival region. The user can recognize the degree of periodontal disease for the tooth and the gingival region to be measured based on the determination result displayed on the display unit 12.

(Overview of Processing Flow of Periodontal Disease Detection System 1)

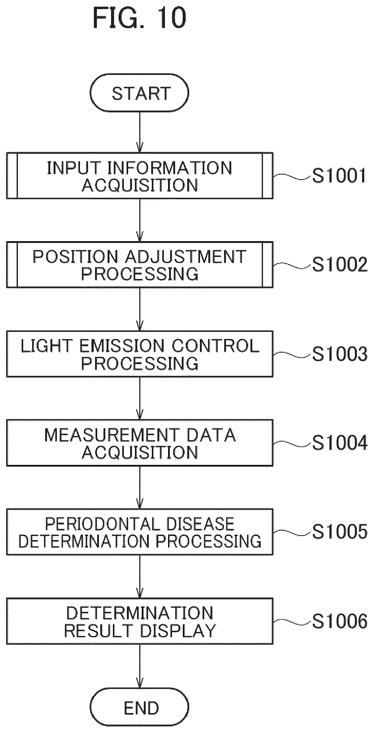

Next, the processing related to periodontal disease detection (periodontal disease detection method) in the periodontal disease detection system 1 will be described based on the flowcharts illustrated in FIGS. 10 to 12. The series of operations of the periodontal disease detection system 1 illustrated in the flowcharts in FIGS. 10 to 12 start when the periodontal disease determination device 100 is activated, and the processing ends when the power is turned off. In the flowcharts illustrated in FIGS. 10 to 12, the processing ends not only when the power is turned off but also when the processing is interrupted. In the following flowcharts, the same contents as those described in the above description of the periodontal disease detection system 1 will not be described or described in a simplified manner.

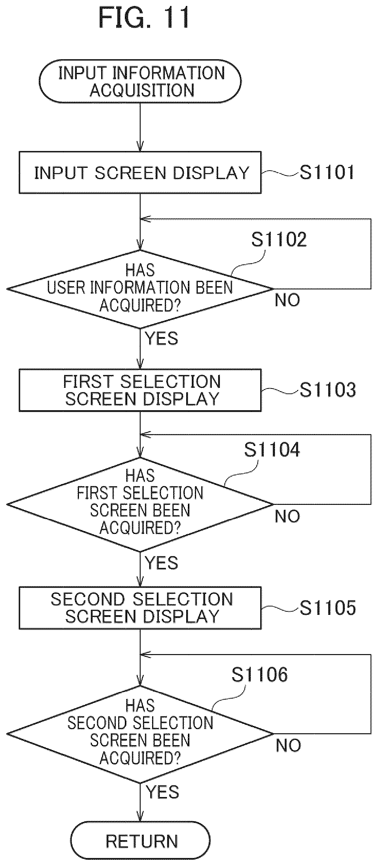

In step S1001 illustrated in FIG. 10, the input information acquisition unit 111 acquires user information inputted by a user, and information on the periodontal part to be measured. Specifically, the input information acquisition unit 111 acquires data inputted by the user to the input screen displayed on the display unit 12 of the user terminal 10. In step S1001, the detailed processing illustrated in FIG. 11 is performed as the processing related to the input information acquisition. The processing related to the input information acquisition in step S1001 will be described with reference to the flowchart illustrated in FIG. 11.

In step S1101 illustrated in FIG. 11, the control unit 110 causes the display unit 12 to display an input screen. Specifically, the display unit 12 displays an input screen of the user information as illustrated in FIG. 6A. Thereafter, the processing proceeds to step S1102.

In step S1102, the input information acquisition unit 111 determines whether the user has acquired the user information inputted via the display unit 12 of the user terminal 10. In step S1102, if the input information acquisition unit 111 determines that the user has acquired the user information inputted via the display unit 12 of the user terminal 10 (step S1102: YES), the processing proceeds to step S1103. However, in step S1102, if the input information acquisition unit 111 determines that the user has not acquired the user information inputted via the display unit 12 of the user terminal 10 (step S1102: NO), the processing in step S1102 is repeated.

In step S1103, the control unit 110 displays a first selection screen. In the present embodiment, the first selection screen is an input screen for acquiring information on the position of a tooth to be measured. The information selected in the first selection screen corresponds to the first selection information. The first selection screen corresponds to the input screen illustrated in FIG. 6B. Thereafter, the processing proceeds to step S1104.

In step S1104, the input information acquisition unit 111 determines whether the user has acquired the first selection information inputted via the display unit 12 of the user terminal 10. In step S1104, if the input information acquisition unit 111 determines that the user has acquired the first selection information inputted via the display unit 12 of the user terminal 10 (step S1104: YES), the processing proceeds to step S1105. However, in step S1104, if the input information acquisition unit 111 determines that the user has not acquired the first selection information inputted via the display unit 12 of the user terminal 10 (step S1104: NO), the processing in step S1104 is repeated.

In step S1105, the control unit 110 displays a second selection screen. In the present embodiment, the second selection screen is an input screen for acquiring information about the gingival region to be measured. The information selected in the second selection screen corresponds to the second selection information. The second selection screen corresponds to the input screen illustrated in FIG. 6C. Thereafter, the processing proceeds to step S1106.

In step S1106, the input information acquisition unit 111 determines whether the user has acquired the second selection information inputted via the display unit 12 of the user terminal 10. In step S1106, if the input information acquisition unit 111 determines that the user has acquired the second selection information inputted via the display unit 12 of the user terminal 10 (step S1106: YES), the processing ends and the processing returns to the flowchart illustrated in FIG. 10. However, in step S1106, if the input information acquisition unit 111 determines that the user has not acquired the second selection information inputted via the display unit 12 of the user terminal 10 (step S1106: NO), the processing in step S1106 is repeated.

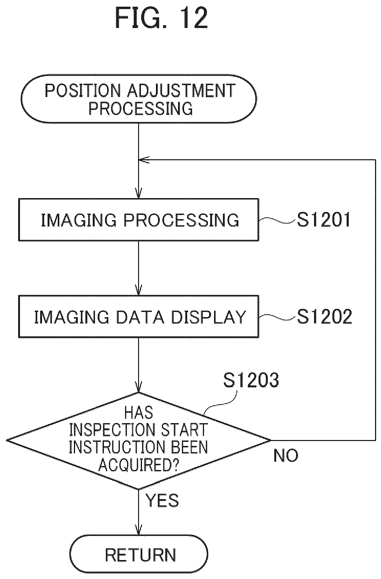

With reference to the flowchart in FIG. 10, further description will be given. In step S1002, the position adjustment unit 112 acquires an image from the measurement device 20 set in the oral cavity by the user, and displays the image on the display unit 12, to allow the user to adjust the position of the measurement device 20. The detailed processing illustrated in FIG. 12 is performed for the position adjustment processing in step S1002. The position adjustment processing in step S1002 will be described with reference to the flowchart illustrated in FIG. 12.

In step S1201 illustrated in FIG. 12, the control unit 110 causes the imaging unit 14 to perform imaging processing and acquires a still image and/or a moving image as imaging data. Thereafter, the processing proceeds to step S1202.

In step S1202, the control unit 110 causes the display unit 12 to display the acquired imaging data. Then, the processing proceeds to step S1203.

In step S1203, the position adjustment unit 112 determines whether or not an inspection start instruction has been acquired. Specifically, in step S1202, the control unit 110 causes the image illustrated in FIG. 7B to be displayed as the imaging data to allow the user to adjust the position of the measurement device 20. Then, the user presses the start button 12a illustrated in FIG. 7B to start the measurement of the oral cavity by the measurement device 20, and the measurement is started. In step S1203, the position adjustment unit 112 determines whether or not the information obtained by the user pressing the start button has been acquired as an inspection start instruction.

In step S1203, if the position adjustment unit 112 determines that the inspection start instruction has been acquired (step S1203: YES), the processing ends and processing returns to the flowchart illustrated in FIG. 10. However, in step S1203, if the position adjustment unit 112 determines that the inspection start instruction has not been acquired (step S1203: NO), the processing returns to step S1201, and the processing from step S1201 is repeated. That is, until the inspection start instruction is given by the user, the position adjustment of the measurement device 20 is performed based on the image captured by the imaging unit 14 and displayed on the display unit 12.

With reference to the flowchart illustrated in FIG. 10, further description will be given. In step S1003, the light emission control unit 113 transmits the light emission control instruction of the light source 210 to the measurement device 20. Specifically, the light emission control unit 113 transmits the light emission control instruction of the light source 210 to the measurement device 20, so that white light is irradiated from the light source 210. Thereafter, the processing proceeds to step S1004.

In step S1004, the measurement data acquisition unit 114 acquires the reflected light acquired by the measurement device 20 as measurement data. The measurement data acquisition unit 114 acquires information on the intensity of the reflected light acquired by the reflected light acquisition unit 220 including a PD (photodiode) as measurement data through the input/output IF 230 and the input/output IF 130. Thereafter, the processing proceeds to step S1005.

In step S1005, the periodontal disease determination unit 115 calculates information indicating the degree of periodontal disease of the user based on the measurement data. Specifically, the periodontal disease determination unit 115 calculates information indicating the degree of periodontal disease based on the measurement data transmitted from the measurement device 20.

In the present embodiment, the periodontal disease determination unit 115 calculates the intensity ratio according to the formula (1) described above. Furthermore, the periodontal disease determination unit 115 determines the presence or absence of periodontal disease and the degree of periodontal disease according to formulas (2) to (5) based on the calculated intensity ratio. Thereafter, the processing proceeds to step S1006.

In step S1006, the determination result display unit 116 displays the determination result determined by the periodontal disease determination unit 115. As described above, FIG. 9 illustrates an example of the determination result of periodontal disease displayed on the display unit 12 of the user terminal 10. As illustrated in FIG. 9, the intensity ratio "2.0", which is the determination result, and the degree of periodontal disease "moderate" are illustrated on the display unit 12 for "LU1" as the tooth, and "Front Middle" as the gingival region, to be measured. The user can recognize the degree of periodontal disease for the tooth and the gingival region to be measured based on the determination result displayed on the display unit 12.

As described above, the periodontal disease detection system 1 according to the present embodiment includes the periodontal disease determination device 100 and the measurement device 20. The periodontal disease determination device 100 includes the position adjustment unit 112 that acquires an image of the measurement device 20 set in the oral cavity by the user from the imaging unit 14, and displays the image on the display unit 12 to allow the user to adjust the position of the measurement device 20. The periodontal disease determination device 100 further includes the light emission control unit 113 that transmits, to the measurement device 20, a light emission control instruction for irradiating light from the light source 210 provided in the measurement device 20. The periodontal disease determination device 100 further includes the measurement data acquisition unit 114 that acquires information on the intensity of the reflected light acquired by the measurement device 20 as measurement data. Furthermore, the periodontal disease determination device 100 includes the periodontal disease determination unit 115 that calculates information indicating the degree of periodontal disease of the user based on the measurement data.

Thus, the user using the periodontal disease detection system 1 can measure the oral cavity by the measurement device 20 while checking the position of the measurement device 20 set in the oral cavity. As a result, the periodontal disease detection system 1 can accurately determine the position of the periodontal part to be detected as periodontal disease.

Further, the light irradiated from the light source 210 has a plurality of wavelengths, and the measurement device 20 may include the reflected light acquisition unit 220 capable of acquiring information on the reflected light having a plurality of wavelengths. Further, the measurement data acquisition part 114 may acquire a plurality of reflected lights acquired by the measurement device 20 as a plurality of pieces of measurement data. Furthermore, the periodontal disease determination unit 115 may calculate an intensity ratio as information indicating the degree of periodontal disease, based on the plurality of pieces of measurement data, and determine the degree of periodontal disease based on the intensity ratio. In this manner, the periodontal disease detection system 1 can more accurately determine the presence or absence of periodontal disease and the degree of progress.

The information on the periodontal part acquired by the input information acquisition unit 111 includes information on a position of a tooth to be measured, and information on gingival regions to be measured. Thus, the periodontal disease detection system 1 can detect the presence or absence of periodontal disease and the progress of periodontal disease in a more detailed region of the periodontal part to be measured.

(Other Embodiments)

The present embodiments have been described above. However, the embodiments are not limited thereto, and various modifications can be made within the scope of the gist of the embodiments. In addition, a new embodiment can be formed by combining parts or all of the various embodiments.

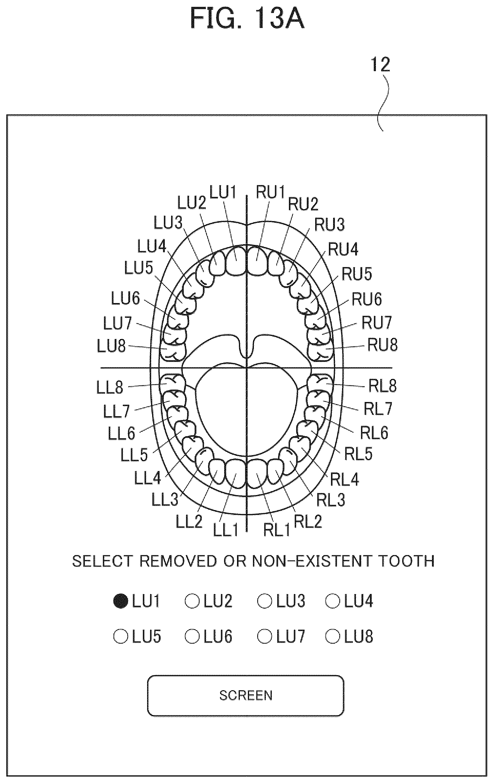

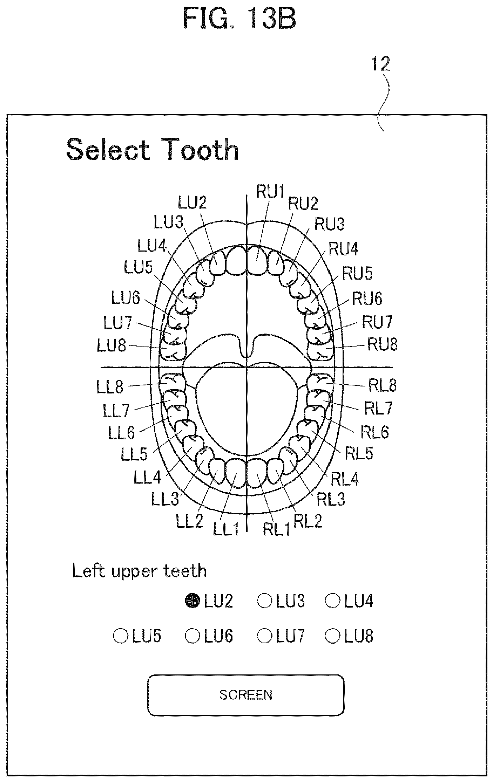

The embodiments described above show, for example, a configuration in which a user selects a tooth to be measured on the input screen corresponding to all teeth illustrated in FIG. 6B. For example, depending on a user, some teeth may not exist due to tooth loss or the like. Therefore, for example, a configuration may be used in which the input information acquisition unit 111 displays the input screen as illustrated in FIGS. 13A and 13B, and registers a tooth of the user that does not exist. That is, the input information acquisition unit 111 acquires information on the tooth that does not exist, and excludes a periodontal part related to the tooth that does not exist from objects to be measured.

For example, in the input screen illustrated in FIG. 13A, the user registers a tooth that does not exist. Then, in the selection screen displayed on the display unit 12, as illustrated in FIG. 13B, for example, a screen is displayed so that the tooth that does not exist cannot be an object to be measured. Thus, the periodontal disease detection system 1 can prevent a measurement error caused by the user mistakenly selecting a tooth that does not exist, and can more accurately determine the position of a periodontal part to be detected as periodontal disease.

In the embodiments described above, the input information acquisition unit 111 acquires user information for one user, but the embodiment is not limited to this configuration. For example, the periodontal disease detection system 1 may be configured as a system corresponding to a plurality of users, and may be configured to identify the user by the "user ID" illustrated in FIG. 5. Furthermore, the user information DB 121 and the measurement data DB 122 of the storage unit 120 may be configured to store the past measurement data of each user. Thus, each user can compare the determination result by the periodontal disease determination device 100 with the result measured and determined in the past, and check an improved or a deteriorated state.

In the embodiments described above, the light source 210 irradiates white light having a plurality of wavelengths, and the plurality of reflected light acquisition units 220 acquire reflected light having a plurality of wavelengths. This configuration does not limit the embodiment. For example, the light source 210 irradiates light of a predetermined wavelength, and the reflected light acquisition unit 220 acquires reflected light of the light of a predetermined wavelength irradiated from the light source 210. In this case, the light source 210 can irradiate light of a plurality of wavelengths by changing the wavelength of the light irradiated from the light source 210 over time divisions. Further, the reflected light acquisition unit 220 adjusts the wavelength in accordance with the light source 210, so that one light receiving element can acquire reflected light of a plurality of lights which is irradiated from the light source 210, and whose wavelengths change over time divisions.

(Supplementary Note)

The above description of the embodiments disclose the following technology.

(Technology 1) A periodontal disease detection system including a periodontal disease determination device and a measurement device, wherein

the periodontal disease determination device comprises:

an input information acquisition unit that acquires information, inputted by a user, on a periodontal part to be measured;
a position adjustment unit that acquires an image of a measurement device set in the oral cavity by the user from an imaging unit, and displaying the image on a display unit to allow the user to adjust the position of the measurement device;
a light emission control unit that transmits to the measurement device a light emission control instruction for irradiating light from a light source provided in the measurement device;
a measurement data acquisition unit that acquires information on an intensity of reflected light acquired by the measurement device as measurement data; and
a periodontal disease determination unit that calculates information indicating the degree of periodontal disease of the user based on the measurement data; and

the measurement device comprises:

the light source that irradiates light based on the light emission control instruction from the light emission control unit; and

a reflected light acquisition unit that acquires reflected light of the irradiated light.

With this configuration, a user using the periodontal disease detection system 1 can measure the oral cavity by the measurement device 20 while checking the position of the measurement device 20 set in the oral cavity. Therefore, the periodontal disease detection system 1 can accurately determine the position of a periodontal part to be detected as periodontal disease.

(Technology 2) The periodontal disease detection system according to technology 1, wherein

the light source irradiates light having a plurality of wavelengths;

the reflected light acquisition unit can acquire information on the reflected light having the plurality of wavelengths;

the measurement data acquisition unit acquires information on the reflected light having the plurality of wavelengths acquired by the measurement device as a plurality of pieces of measurement data; and

the periodontal disease determination unit calculates an intensity ratio as information indicating the degree of periodontal disease, based on the plurality of pieces of measurement data.

The periodontal disease determination unit 115 of the periodontal disease determination device 100 determines periodontal disease based on the intensity of the reflected light having the plurality of wavelengths. Therefore, the periodontal disease detection system 1 can more accurately determine the presence or absence of periodontal disease and the degree of progress.

(Technology 3) The periodontal disease detection system according to Technology 1 or 2, in which the input information acquisition unit acquires information on a tooth that does not exist, and excludes a periodontal part related to the tooth that does not exist from objects to be measured.

With this configuration, the periodontal disease detection system 1 excludes the measurement on a tooth that does not exist, and performs the measurement of existing teeth and their gingival regions. Thus, the periodontal disease detection system 1 can prevent a measurement error caused by the user mistakenly selecting a tooth that does not exist, and can more accurately determine the position of a periodontal part to be detected as periodontal disease.

(Technology 4) The periodontal disease detection system according to any one of technologies 1 to 3, wherein the information on the periodontal part includes information on a position of a tooth to be measured, and information on gingival regions to be measured.

With this configuration, the periodontal disease detection system 1 irradiates light (white light) from a light source 210 to a specific tooth or gingival region, acquires the reflected light, and determines periodontal disease in the periodontal disease determination device 100. Therefore, the periodontal disease detection system 1 can detect the presence or absence of periodontal disease and the progress of periodontal disease in a more detailed region of the periodontal part to be measured.

(Technology 5) A periodontal disease detection method executed by a computer, including:

acquiring information, inputted by a user, on a periodontal part to be measured;

acquiring an image of a measurement device set in the oral cavity by the user to allow the user to adjust the position of the measurement device;

displaying the image on a display unit;

transmitting to the measurement device a light emission control instruction for irradiating light from a light source provided in the measurement device;

acquiring information on an intensity of reflected light, which is irradiated from the light source and is acquired by a reflected light acquisition unit provided in the measurement device, as measurement data; and

calculating information indicating the degree of periodontal disease of the user based on the measurement data.

With this configuration, the user using the periodontal disease detection method can measure the oral cavity by the measurement device 20 while checking the position of the measurement device 20 set in the oral cavity. Therefore, the periodontal disease detection method can accurately define the position of the periodontal part to be detected.

(Technology 6): A periodontal disease detection program that causes a computer to execute:

acquiring information, inputted by a user, on a periodontal part to be measured;

acquiring an image of a measurement device set in the oral cavity by the user to allow the user to adjust the position of the measurement device;

displaying the image on a display unit;

transmitting to the measurement device a light emission control instruction for irradiating light from a light source provided in the measurement device;

acquiring information on an intensity of reflected light, which is irradiated from the light source and is acquired by a reflected light acquisition unit provided in the measurement device, as measurement data; and

calculating information indicating the degree of periodontal disease of the user based on the measurement data.

With this configuration, the user using the periodontal disease detection program can measure the oral cavity by the measurement device 20 while checking the position of the measurement device 20 set in the oral cavity. Therefore, the periodontal disease detection program can accurately determine the position of the periodontal part to be detected.

The entire content of Japanese Patent Application No. 2022-189573 (filed on November 28, 2022) is incorporated herein by reference.

The contents of the present disclosure have been described in accordance with the embodiments. However, it is obvious to those skilled in the art that the present disclosure is not limited to these descriptions and that various modifications and improvements can be made.

(Industrial Applicability)

The present disclosure is applicable to a periodontal disease detection system that can accurately determine the position of the periodontal part to be detected. Specifically, the present disclosure is applicable to periodontal disease detection devices for commercial use and home use.

REFERENCE SIGNS LIST

    1 Periodontal disease detection system
    10 User terminal
    12 Display unit
    14 Imaging unit
    20 Measurement device
    100 Periodontal disease determination device
    110 Control unit
    111 Input information acquisition unit
    112 Position adjustment unit
    113 Light emission control unit
    114 Measurement data acquisition unit
    115 Periodontal disease judgment unit
    116 Judgment result display unit
    120 Storage unit
    130, 230 Input/output IF
    140 Communication IF
    150 Display IF
    160 Camera IF
    200 Sensor
    210 Light source
    220, 220a, 220b Reflected light acquisition unit

**Claims**

1. A periodontal disease detection system comprising a periodontal disease determination device and a measurement device, wherein

the periodontal disease determination device comprises:

    an input information acquisition unit that acquires information, inputted by a user, on a periodontal part to be measured;
    a position adjustment unit that acquires an image of the measurement device set in the oral cavity by the user from an imaging unit, and displaying the image on a display unit, so that the user can adjust the position of the measurement device;
    a light emission control unit that transmits to the measurement device a light emission control instruction for irradiating light from a light source provided in the measurement device;
    a measurement data acquisition unit that acquires information on an intensity of reflected light acquired by the measurement device as measurement data; and
    a periodontal disease determination unit that calculates information indicating the degree of periodontal disease of the user based on the measurement data; and

the measurement device comprises:

    the light source that irradiates light based on the light emission control instruction from the light emission control unit; and
    a reflected light acquisition unit that acquires reflected light of the irradiated light.

2. The periodontal disease detection system according to claim 1, wherein

    the light source irradiates light having a plurality of wavelengths;
    the reflected light acquisition unit can acquire information on the reflected light having the plurality of wavelengths;
    the measurement data acquisition unit acquires information on the reflected light having the plurality of wavelengths acquired by the measurement device as a plurality of pieces of measurement data; and
    the periodontal disease determination unit calculates an intensity ratio as information indicating the degree of periodontal disease, based on the plurality of pieces of measurement data.

3. The periodontal disease detection system according to claim 1 or 2, wherein the input information acquisition unit acquires information on a tooth that does not exist, and excludes a periodontal part related to the tooth that does not exist from objects to be measured.

4.  The periodontal disease detection system according to any one of claims 1 to 3, wherein the information on the periodontal part includes information on a position of a tooth to be measured, and information on gingival regions to be measured.

5.  A periodontal disease detection method executed by a computer, comprising:

    acquiring information, inputted by a user, on a periodontal part to be measured;
    acquiring an image of a measurement device set in the oral cavity by the user, so that the user can adjust the position of the measurement device;
    displaying the image on a display unit;
    transmitting to the measurement device a light emission control instruction for irradiating light from a light source provided in the measurement device;
    acquiring information on an intensity of reflected light, which is irradiated from the light source and is acquired by a reflected light acquisition unit provided in the measurement device, as measurement data; and
    calculating information indicating the degree of periodontal disease of the user based on the measurement data.

6.  A periodontal disease detection program that causes a computer to execute:

    acquiring information, inputted by a user, on a periodontal part to be measured;
    acquiring an image of a measurement device set in the oral cavity by the user to allow the user to adjust the position of the measurement device;
    displaying the image on a display unit;
    transmitting to the measurement device a light emission control instruction for irradiating light from a light source provided in the measurement device;
    acquiring information on an intensity of reflected light, which is irradiated from the light source and is acquired by a reflected light acquisition unit provided in the measurement device, as measurement data; and
    calculating information indicating the degree of periodontal disease of the user based on the measurement data.

# FIG. 1

# FIG. 2

# FIG. 3A

# FIG. 3B

# FIG. 4

100

110                                         120

## CONTROL UNIT

111
INPUT INFORMATION
ACQUISITION UNIT

112
POSITION ADJUSTMENT
UNIT

113
LIGHT EMISSION
CONTROL UNIT

114
MEASUREMENT DATA
ACQUISITION UNIT

115
PERIODONTAL DISEASE
DETERMINATION UNIT

116
DETERMINATION
RESULT DISPLAY UNIT

## STORAGE UNIT

121
USER
INFORMATION
DB

122
MEASUREMENT
DATA DB

# FIG. 5

| USER NAME | name |
|---|---|
| USER ID | U001 |
| AGE | 30 |

| | | | GINGIVAL REGION | LU8 | LU7 | LU6 | LU5 | LU4 | LU3 | LU2 | LU1 | RU1 | RU2 | RU3 | RU4 | RU5 | RU6 | RU7 | RU8 |
|---|---|---|---|---|---|---|---|---|---|---|---|---|---|---|---|---|---|---|---|
| | | | | LEFT SIDE | | | | | | | | RIGHT SIDE | | | | | | | |
| UPPER JAW | UPPER ROW : OBJECTS TO BE MEASURED LOWER ROW : DETERMINATION RESULT | Front | Distal | | | | | | | | | | | | | | | | |
| | | Front | Middle | | | | | | | | ○ MODERATE | | | | | | | | |
| | | Front | Mesial | | | | | | | | | | | | | | | | |
| | | Back | Middle | | | | | | | | | | | | | | | | |

| | | | GINGIVAL REGION | LL8 | LL7 | LL6 | LL5 | LL4 | LL3 | LL2 | LL1 | RL1 | RL2 | RL3 | RL4 | RL5 | RL6 | RL7 | RL8 |
|---|---|---|---|---|---|---|---|---|---|---|---|---|---|---|---|---|---|---|---|
| LOWER JAW | UPPER COLUMN : OBJECTS TO BE MEASURED LOWER COLUMN : DETERMINATION RESULT | Front | Distal | | | | | | | | | | | | | | | | |
| | | Front | Middle | | | | | | | | | | | | | | | | |
| | | Front | Mesial | | | | | | | | | | | | | | | | |
| | | Back | Middle | | | | | | | | | | | | | | | | |

EP 4 628 025 A1

# FIG. 6A

14

10

12

User Information

User Details

Name

Date Of Birth

GENDER

ADD USER

# FIG. 6B

Select Tooth

Left upper teeth

● LU1  ○ LU2  ○ LU3  ○ LU4
○ LU5  ○ LU6  ○ LU7  ○ LU8

SCREEN

# FIG. 6C

Select Point

Front Distal

Front Mesial

Back Middle

Front Middle

○ Front Distal   ○ Front Mesial
○ Back Middle   ○ Front Middle

SCREEN

# FIG. 6D

# FIG. 6E

# FIG. 7A

Position the device

OK,GOT IT!

# FIG. 7B

POSITION ADJUSTMENT PROCESSING

# FIG. 8

# FIG. 9

Results        SAVE

← DETERMINATION RESULT DISPLAY

LU1

■ Point : Front  Middle

2.0

■ Degree of Periodontal Disease:
Moderate

SCREEN  ANOTHER  TOOTH

GENERATE  REPORT

# FIG. 10

START

INPUT INFORMATION ACQUISITION ──S1001

POSITION ADJUSTMENT PROCESSING ──S1002

LIGHT EMISSION CONTROL PROCESSING ──S1003

MEASUREMENT DATA ACQUISITION ──S1004

PERIODONTAL DISEASE DETERMINATION PROCESSING ──S1005

DETERMINATION RESULT DISPLAY ──S1006

END

# FIG. 11

INPUT INFORMATION
ACQUISITION

INPUT SCREEN DISPLAY — S1101

S1102

HAS
USER INFORMATION BEEN
ACQUIRED?    NO

YES

FIRST SELECTION
SCREEN DISPLAY — S1103

S1104

HAS
FIRST SELECTION
SCREEN BEEN
ACQUIRED?    NO

YES

SECOND SELECTION
SCREEN DISPLAY — S1105

S1106

HAS
SECOND SELECTION
SCREEN BEEN
ACQUIRED?    NO

YES

RETURN

29

# FIG. 12

POSITION ADJUSTMENT PROCESSING

IMAGING PROCESSING — S1201

IMAGING DATA DISPLAY — S1202

S1203

HAS INSPECTION START INSTRUCTION BEEN ACQUIRED?  NO

YES

RETURN

# FIG. 13A

12

SELECT REMOVED OR NON-EXISTENT TOOTH

●LU1  ○LU2  ○LU3  ○LU4

○LU5  ○LU6  ○LU7  ○LU8

SCREEN

# FIG. 13B

12

Select Tooth

RU1
RU2
LU2
LU3
RU3
LU4
RU4
LU5
RU5
LU6
RU6
LU7
RU7
LU8
RU8

LL8
RL8
LL7
RL7
LL6
RL6
LL5
RL5
LL4
RL4
LL3
RL3
LL2
RL2
LL1 RL1

Left upper teeth

● LU2    ○ LU3    ○ LU4

○ LU5    ○ LU6    ○ LU7    ○ LU8

SCREEN

## INTERNATIONAL SEARCH REPORT

| International application No. |
| --- |
| **PCT/JP2023/040358** |

| A. | CLASSIFICATION OF SUBJECT MATTER |
| --- | --- |

*A61B 10/00*(2006.01)i; *A61B 1/24*(2006.01)i; *A61C 19/04*(2006.01)i
FI:   A61B10/00 E; A61B1/24; A61C19/04 Z

According to International Patent Classification (IPC) or to both national classification and IPC

| B. | FIELDS SEARCHED |
| --- | --- |

Minimum documentation searched (classification system followed by classification symbols)

A61B10/00; A61B1/24; A61C19/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | JP 2021-505304 A (KONINKLIJKE PHILIPS NV) 18 February 2021 (2021-02-18) abstract, paragraphs [0028]-[0029], [0060], fig. 4 | 1-6 |
| A | WO 2016/076140 A1 (SONY CORP) 19 May 2016 (2016-05-19) abstract, paragraphs [0024]-[0029], [0139]-[0144], fig. 1, 19 | 1-6 |
| A | JP 2012-143528 A (ADVANCE CO LTD) 02 August 2012 (2012-08-02) abstract, paragraph [0031] | 1-6 |
| A | JP 2016-535654 A (CARESTREAM HEALTH INC) 17 November 2016 (2016-11-17) paragraph [0035] | 1-6 |
| A | JP 2013-248220 A (SONY CORP) 12 December 2013 (2013-12-12) abstract, paragraph [0035], fig. 1-2 | 1-6 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **15 January 2024** | **30 January 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

# EP 4 628 025 A1

<table>
<tr><td colspan="2" align="center">INTERNATIONAL SEARCH REPORT<br>Information on patent family members</td><td colspan="2">International application No.<br><br>**PCT/JP2023/040358**</td></tr>
</table>

| Patent document cited in search report | Publication date (day/month/year) | Patent family member(s) | Publication date (day/month/year) |
|---|---|---|---|
| JP 2021-505304 A | 18 February 2021 | US 2021/0161391 A1<br>abstract, paragraphs [0034]-[0035], [0066], fig. 4<br>WO 2019/115201 A1<br>CN 111479498 A | |
| WO 2016/076140 A1 | 19 May 2016 | US 2017/0319065 A1<br>abstract, paragraphs [0072]-[0077], [0191]-[0199], fig. 1, 19<br>EP 3219250 A1<br>KR 10-2017-0086024 A1<br>CN 107106018 A1 | |
| JP 2012-143528 A | 02 August 2012 | US 2013/0286174 A1<br>abstract, paragraph [0140]<br>WO 2012/096312 A1<br>EP 2664272 A1<br>CN 103347436 A | |
| JP 2016-535654 A | 17 November 2016 | US 2016/0270716 A1<br>paragraph [0046]<br>WO 2015/069704 A1 | |
| JP 2013-248220 A | 12 December 2013 | US 2013/0323674 A1<br>abstract, paragraphs [0084]-[0085], fig. 1-2<br>CN 103445761 A | |

Form PCT/ISA/210 (patent family annex) (July 2022)

34

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015069704 A

- JP 2022189573 A

**Non-patent literature cited in the description**

- **PRASANTH CHANDRA SEKHAR et al.** Discrimination of periodontal diseases using diffuse reflectance spectral intensity ratios. *Journal of Biomedical Optics*, February 2012, vol. 17 (2), 027001-1, 027001-9